# EUROPEAN PATENT APPLICATION

(11) **EP 1 510 211 A1**
(43) Date of publication of application: **02.03.2005**
(21) Application number: 03019310.6
(22) Date of filing: 26.08.2003
(51) Int. Cl.: A61K 31/44, A61K 31/685, A61P 35/00, A61P 17/06

(54) **Pharmaceutical composition comprising N-retinyl-N-retinylidene Ethanolamine (A2E), isoforms of A2E or derivatives thereof**

(71) Applicant: Lynkeus Biotech GmbH, 97076 Würzburg (DE)
(72) Inventor: The designation of the inventor has not yet been filed
(74) Representative: VOSSIUS & PARTNER

(57) **Abstract**

The present invention relates to a pharmaceutical composition comprising N-retinyl-N-retinylidene ethanolamine (A2E), isoforms of A2E or derivatives thereof which induce apoptosis by detaching pro-apoptotic proteins from mitochondria. Further, an antidote for said pharmaceutical composition is disclosed. The invention further relates to a use of N-retinyl-N-retinylidene ethanolamine (A2E), isoforms of A2E or derivatives for the preparation of a pharmaceutical composition and the use of phospholipids for the preparation of an antidote for said pharmaceutical composition. Moreover a method for the treatment of proliferative diseases and a method of screening for isoforms of A2E or derivatives thereof which induce apoptosis by detaching pro-apoptotic proteins from mitochondria are disclosed.

## Description

The present invention relates to a pharmaceutical composition comprising N-retinyl-N-retinylidene ethanolamine (A2E), isoforms of A2E or derivatives thereof which induce apoptosis by detaching pro-apoptotic proteins from mitochondria. Further, an antidote for said pharmaceutical composition is disclosed. The invention further relates to a use of N-retinyl-N-retinylidene ethanolamine (A2E), isoforms of A2E or derivatives for the preparation of a pharmaceutical composition and the use of phospholipids for the preparation of an antidote for said pharmaceutical composition. Moreover a method for the treatment of proliferative diseases and a method of screening for isoforms of A2E or derivatives thereof which induce apoptosis by detaching pro-apoptotic proteins from mitochondria are disclosed.

A variety of documents is cited throughout this specification. The disclosure content of said documents including manufacturer's manuals is herewith incorporated by reference in its entirety.

The human retina is a multilayered tissue composed of a number of distinct cell types that are highly specialized in their function to transform light energy into electric impulses and to further transmit these signals to the brain where they are processed and perceived as vision. An essential prerequisite for these processes is a strong metabolic activity. Active supply of photoreceptors with nutrient substances from the blood circulation is mediated by the retinal pigment epithelium (RPE), as well as the simultaneous removal and processing of degradation products of the visual cascade. The initiation of the cascade of events which link the incoming light to the sensation of "vision" occurs at the level of the photoreceptors in the retina. Recent findings have shown, that the photoreceptors are particular susceptible to damaging light leading to cell death and thus resulting in visual morbidity. The accumulation of the autofluorescent age pigment lipofuscin in RPE cell phagolysosomes and the formation of so-called drusen and other deposits in the region of Bruch's membrane, a thin sheet of extracellular proteins and polysaccharides which separates the RPE from the underlying choroidal blood vessels are also considered as initial steps in the degeneration process of photoreceptor cells.

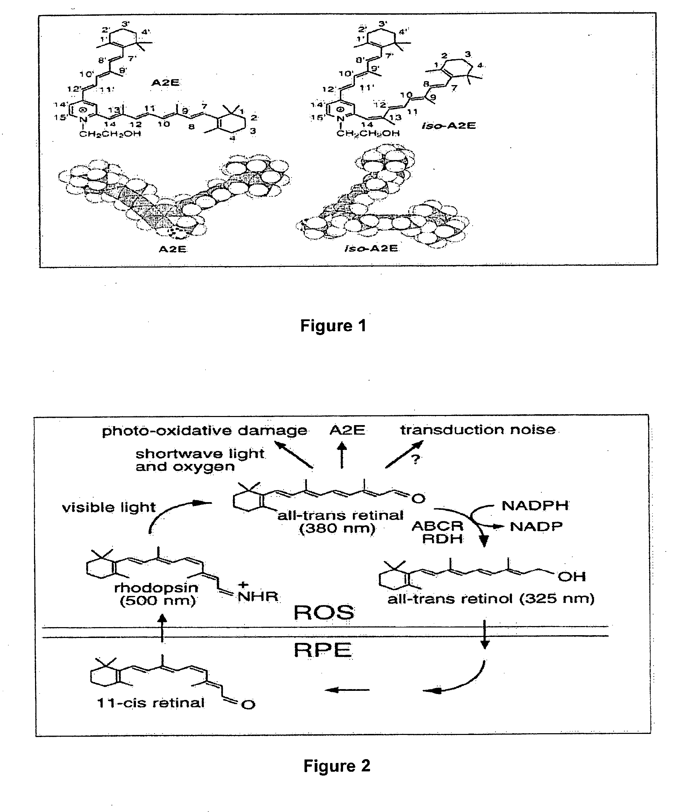

Accumulated lipofuscin in phagolysosomes of retinal pigment epithelium (RPE) cells in the eye harbours two unusual retinoids, the lipophilic cations N-retinyl-N-retinylidene ethanolamine (A2E) and its isoform, *iso-A2E* (Eldred and Lasky, 1993; Parish et al., 1998) (for their structures, see Fig. 1). The compounds can be synthesized *in vitro* from 2 molecules of retinal and 1 molecule of ethanolamine. The precursors are present in photoreceptor outer segment membranes, where 11-cis-retinal serves as the chromophore of the visual pigment rhodopsin, and phosphatidylethanolamine is an abundant membrane phospholipid. *In vivo* A2E is formed as by-product of the visual cycle (see Fig. 2) presumably from 2 molecules of retinal and 1 molecule of phosphatidylethanolamine (Mata et al., 2000).

Apoptosis (programmed cell death) is a form of cell death that is accomplished by a special cellular machinery (Hengartner, 2000). Mitochondria have a central role in apoptosis (Richter, 1993) because they respond to apoptosis-signaling molecules and release apoptosis-inducing factors (Green and Reed, 1998). Animal models of retinal diseases revealed that photoreceptors can die apoptotically (Remé *et al*., 1998), but until recently it was not clear how apoptosis in the retina is induced. Now at least two low molecular weight-compounds, retinal and A2E (see above), and three proteins (rhodopsin, the Rim protein, and the Rpe65 protein) are known to be intimately involved in the apoptosis of RPE cells.

The respiratory chain, which transports electrons and protons, is located in the inner mitochondrial membrane and is composed of four complexes (I-IV). At the level of complex IV, which is identical with cytochrome c oxidase, mitochondria reduce molecular oxygen to water and thereby convert energy. This energy is used to produce ATP and to transport molecules across the inner mitochondrial membrane. Until recently mitochondria were exclusively viewed as life-supporting organelles.

A few years ago it was realized that mitochondria also actively participate in the demise of cells because they are engaged in the execution of apoptosis (Richter, 1993; Hengartner, 2000). Mitochondria contain proteins which, when released into the cytosol, help to selectively destroy vital components of the cell such as enzymes and DNA. Presently four such proteins have been identified: Cytochrome c, apoptosis inducing factor (AIF), SMAC/Diablo, and endonuclease D.

During the last years the view on proliferative diseases has been shifted from describing said diseases to be the result of uncontrolled proliferation to describing said diseases to be the result of an absence of apoptosis in affected cells and diseases.

A large number of compounds is known in the art which have the ability to inhibit the proliferation of pathologically proliferating cells. However, it is also known in the art, that, dependent from the particular route of administration, the administration of such compounds is accompanied with different kinds of adverse side effects. This is particularly described for the systemic administration. Said strategies for the treatment and healing of proliferative diseases make use of a fundamental effect of the active compounds on the regulatory mechanisms of the proliferation of cells. The majority of compounds affects all different kinds of proliferating cells without any discrimination between non-pathological cells and pathologically deregulated cells. Consequently, it is assessed for every single case whether the risk of such treatment is in an acceptable balance with the expected benefit.

Thus, the technical problem underlying the present invention was to provide alternative means and methods for the for the treatment of proliferative diseases. Such alternative means and methods should inherently reduce or even abolish the risks associated with the administration of the means in favor of exclusive or preferential targeting of pathologically deregulated cells.

The solution to said technical problem is achieved by providing the embodiments characterized in the claims.

Accordingly, the present invention relates to a pharmaceutical composition comprising N-retinyl-N-retinylidene ethanolamine (A2E), isoforms of A2E or derivatives thereof which induce apoptosis by detaching pro-apoptotic proteins from mitochondria and optionally further comprising suitable formulations of carriers, stabilizers and/or excipients.
The term "isoforms of A2E or derivatives thereof" as used herein relates to molecules of the family of A2E which show common characteristics in the molecular structure and in their ability to induce apoptosis by detaching pro-apoptotic proteins from mitochondria. One example for such isoform of A2E is depicted in the appended figure 1. Derivatives of A2E or isoforms of A2E can be obtained e.g. by chemical synthesis.
The ability of isoforms of A2E or derivatives thereof to induce apoptosis by detaching pro-apoptotic proteins from mitochondria may be tested by a method comprising the step of contacting cells, e.g. a porcine primary retinal pigment epithelial cells (RPE) or cell lines e.g. selected from HeLa cells (see e.g. ATCC No. CCL-2 and CCL-2.2), ARPE-19 cells (see e.g. ATCC No. CRL-2302)and MDCK-F1 cells (see e.g. Schar-Zammaretti et al. Anal Chem. 2002; 74(16):4269-74) with an isoform of A2E or derivative of A2E or an isoform thereof and detecting whether said contacting results in the induction of apoptosis in said cell. Different methods for the detection of apoptosis are known in the art (see e.g. Dulat et al. Eur J Immunol. 2001;31(7):2217-26). One example for a corresponding assay is described in the appended examples. Examples of suitable pharmaceutical carriers are well known in the art and include phosphate buffered saline solutions, water, emulsions, such as oil/water emulsions, various types of wetting agents, sterile solutions, etc. Compositions comprising such carriers can be formulated by conventional methods.
Examples for pharmaceutical acceptable stabilizers and/or excipients are known to the person skilled in the art of galenic and formulation of pharmaceutical compositions. Furthermore, suitable examples are given in handbooks, such as Rowe et al., Handbook of Pharmaceutical Excipients Pharmaceutical Press.
The pharmaceutical composition of the invention can be administered to the subject at a suitable dose. Administration of the suitable compositions may be effected by different ways, e.g., by intravenous, intraperitoneal, subcutaneous, intramuscular, parenteral, topical or intradermal administration; particularly preferred routes of administration for the pharmaceutical composition of the invention are described herein below. Said ways of administration particularly comprise the perfusion of specific tissues and organs prior, during or after surgical isolation of said specific tissues and organs. The dosage regiment will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Generally, the regimen as a regular administration of the pharmaceutical composition should be in the range of 0,1 µg to 10 mg units per day. If the regimen is a continuous infusion, it should also be in the range of 0,1 µg to 10 mg units per kilogram of body weight per minute, respectively. However, a more preferred dosage for continuous infusion might be in the range of 0.001 µg to 10 mg units per kilogram of body weight per hour. Progress can be monitored by periodic assessment. For non-systemic administration of the pharmaceutical composition of the invention, e.g. for local administration, the dosage may be adapted to achieve corresponding concentrations in the target cells, target tissue or target organ. The compositions of the invention may be administered locally or systematically. However, it is of note that the pharmaceutical composition of the invention comprising A2E may be administered locally only. Pharmaceutical compositions of the invention comprising isoforms of A2E or derivatives thereof, which have to be transformed from a non-apoptosis-inducing pro-form to apoptosis-inducing compound by electromagnetic irradiation may be administered systemically and non-systemically.
For local administration of the pharmaceutical composition of the invention into a target site the composition may be delivered e.g. by catheter to a site in an artery. Preparations for parenteral administration particularly comprise injections, infusions and salves. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present invention might comprise proteinaceous carriers, like, e.g., serum albumin or immunoglobulin, preferably of human origin. For the prolongation of the shelf live of the pharmaceutical composition of the invention the composition may be lyophilized. Prior to its administration said lyophilized formulation of the composition may require a reconstitution.
The pharmaceutical composition of the invention may be administered once or repeatedly in intervals of hours, days, weeks of month. The interval for the repeatedly administration of the pharmaceutical composition of the invention may depend from the specific schema for the application developed by a physician for a patient in the need thereof.
Furthermore, it is envisaged that the pharmaceutical composition of the invention might comprise further biologically active agents, depending on the intended use of the pharmaceutical composition. Such agents might be drugs acting on the gastrointestinal system, drugs acting as cytostatica, drugs preventing hyperurikemia and/or agents such as T-cell stimulatory/co-stimulatory molecules or cytokines known in the art.
Possible indications for administration of the pharmaceutical composition of the invention are proliferative diseases such as psoriasis or tumorous diseases. Particularly preferred are diseases of the skin. Tumorous diseases especially comprise cancer diseases of the skin such as malignant melonoma. Also envisaged are epithelial cancers/carcinomas such as mamma carcinoma, colon carcinoma, prostate carcinoma, ovarial carcinoma or lung carcinoma or other tumorous diseases like haematological tumors, gliomas, sarcomas or osteosarcomas.
As detailed herein, the pharmaceutical composition of the invention may be administered to a patient in need of medical intervention (preferably a human patient). The pharmaceutical composition may administered alone or in combination with other medicaments/pharmaceutical compositions. These further medicaments/pharmaceutical compositions may be administered simultaneously or non-simultaneously with the pharmaceutical composition of the invention.

It has been surprisingly found in accordance with the present invention, that A2E, isoforms of A2E or derivatives thereof are suitable for the induction of apoptosis in cells which are affected by proliferative diseases. In different experimental approaches it has been demonstrated that the composition of the invention allows a target directed elimination of proliferating cells.
For example, the local administration of a pharmaceutical composition of the invention comprising A2E by direct application into tumor tissue results in the elimination of said tumor tissue by apoptosis. Due to the fact that the eliminated cells undergo apoptosis instead of a non-programmed cell death said elimination of tumor cells is not accompanied by inflammatory reactions. In particular, the cells are eliminated by a specific targeting of the cytochrome c-oxidase VI complex. Due to said targeting there will be no development of resistance in tumor tissue, since the structure of said complex is essential and highly conserved. Modifications in said complex are according to the state of the art not tolerated and, thus, can not be transferred to a next generation of cells.

In one embodiment of the pharmaceutical composition of invention the said A2E, isoforms of A2E or derivatives thereof is/are linked to a ligand which specifically binds to/interacts with a cell surface molecule, a molecule in the cytosol of a cell or a molecule specific for mitochondria.
The linkage between said A2E, isoforms of A2E or derivatives thereof and said ligand is in line with the present invention preferably a chemical bond, more preferred a covalent bond.
The term "ligand" defines in the context of the present invention a molecule which specifically binds to/interacts with a specific "receptor". (Poly)peptide/protein ligands as well as (poly)peptide/protein receptors are particularly envisaged by the invention. Preferred ligands comprise antibodies, antibody fragments or derivatives thereof. Said antibody fragments are known in the art and comprise, but are not limited to, Fab-fragments, F(ab₂)' fragments, Fv fragments and the like. Derivatives include scFv's, chimeric or humanized antibodies. Further examples for ligands in line with the present invention are aptamers. In accordance with the present invention, the term "aptamer" means nucleic acid molecules that can bind to target molecules by means of their three-dimensional structure and not by hybridization. Aptamers commonly comprise RNA, single stranded DNA, modified RNA or modified DNA molecules. The preparation of aptamers is well known in the art and may involve, inter alia, the use of combinatorial RNA libraries to identify binding sides (Gold, Ann. Rev. Biochem. 64 (1995), 763-797).
The binding/interaction of the ligand/receptor pair may be characterized as the binding/interaction of an antigen-interaction-site to/with its antigen.
The term "antigen-interaction-site" defines, in accordance with the present invention, a motive, preferably of a polypeptide, which shows the capacity of specific interaction with a specific antigen or a specific group of antigens. The "interaction" of said "antigen-interaction-site" with an antigen is specific and characterized, in preferred embodiments, by a high binding constant of ≤ 10⁻⁹M. In contrast, an unspecific interaction with an antigen is characterized by an extremely low binding constant of ≥ 10⁻⁵M. The specific interaction of the antigen-interaction-site with its specific antigen may result in an initiation of a signal, e.g. due to the induction of a change of the conformation of the antigen, an oligomerization of the antigen, etc. Said binding may be exemplified by the specificity of a "key-lock-principle". Thus, specific motives in the amino acid sequence of the antigen-interaction-site and the antigen bind to each other as a result of their primary, secondary or tertiary structure as well as the result of secondary modifications of said structure. The specific interaction of the antigen-interaction-site with its specific antigen may result as well in a simple binding of said site to the antigen.
Examples for the specific interaction of an antigen-interaction-site with a specific antigen comprise the specificity of a soluble ligand for its specific membrane bound receptor. Said definition particularly comprises the interaction of ligands which induce a signal upon binding to its specific receptor. Examples for corresponding ligands comprise cytokines which interact with /bind to its specific cytokine-receptors. Also particularly comprised by said definition is the binding of an antigen-interaction-site to antigens like antigens of the selectin family, integrins and of the family of growth factors like EGF. An other example for said interaction, which is also particularly comprised by said definition, is the interaction of an antigenic determinant (epitope) with the antigenic binding site of an antibody.

The term "cell surface molecule" as used herein denotes molecules which are presented on and/or attached to the surface of a cell. Examples for said cell surface molecules are membrane and transmembrane proteins (including modified variants, such as glycosylated variants), molecules attached to said proteins or the cell surface as well as glycosylated moieties such as for example lipids. Attachment is to be preferably understood as being effected by way of an integral membrane protein, a GPI-linked (glycosyl phosphatidyl inositol-linked) protein, a proteinaceous or non-proteinaceous moiety bound covalently or non-covalently to another carrier molecule such as sugar moieties or ganglioside moieties. Preferably, said cell surface molecule is a tumor-specific marker. A tumor-specific marker is a tumor-associated cell surface antigen which is either found exclusively on tumor cells, is overexpressed on tumor cells as compared to non-malignant cells or which is expressed by tumor and non-tumor cells but accessibly presented to the immune system only by tumor cells.
Tumor-associated cell surface antigens can be expressed not only on tumor cells but also on cells/tissue which are/is not essential for survival or which can be replenished by stem cells not expressing tumor-associated cell surface antigen. Furthermore, tumor-associated cell surface antigen can be expressed on malignant cells and non-malignant cells but is better accessible by a therapeutic agent of interest on malignant cells. Examples of over-expressed tumor-associated cell surface antigens are EGF-Receptor, Her2/neu, Her-3 and Her-4. An example of a tumor-associated cell surface antigen which is tumor specific is EGFRV-III. An example of a tumor-associated cell surface antigen which is presented on a cell which is non-essential for survival is PSMA. Examples of tumor-associated cell surface antigens which are presented on cells which are replenished are CD19, CD20 and CD33. An example of a tumor-associated cell surface antigen which is better accessible in a malignant state than in a non-malignant state is EpCAM.
The terms "molecule in the cytosol of a cell" and "molecule specific for mitochondria" define in the context of the present invention molecules which are characteristic for said cellular compartments, such as that they specifically or preferentially occur in or are associated with these compartments. The molecules consequently represent target structures which may allow to direct the active compound of the pharmaceutical composition of the invention to the specific compartment subsequent of the uptake of the linked molecule by a cell.

As indicated above, the recited cell surface molecule may be in accordance with the invention a tumor marker, tumor associated antigen and/or a molecule which is expressed by tumor and non-tumor cells but accessibly presented to the immune system only by tumor cells.
Preferably, said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

It is particularly preferred for the pharmaceutical composition of the invention that said A2E, isoforms of A2E or derivatives thereof and said ligand are linked by a covalent bond.

It is further envisaged by the present invention that the pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.

It has been surprisingly found in accordance with the present invention, that isoforms of A2E or derivatives thereof exist in a pro-form of the active compound of the invention. After transformation of said pro-form to the active form said isoforms of A2E or derivatives thereof achieve the capacity to induce apoptosis by detaching pro-apoptotic proteins from mitochondria. The transformation may be effected electromagnetic irradiation. This surprising technical feature of corresponding compounds allows the systemic application of an agent which is harmless to all cells, tissues and organs of an individual until it is transformed from its pro-form to its active-form in a particularly limited target area.
For example, after systemic administration by infusion or after local, e.g. topical, administration of the pro-form of the active compound a limited target area which is effected by a locally limited disease, may treated and/or cured by transforming said pro-form by irradiation of the locally limited area with light. Due to said irradiation the pro-form is transformed into the active-form only in the target area, whereas the pro-form remains untransformed and harmless in all other areas which are not irradiated.
The transformation by irradiation with blue light is effected e.g. by photo-isomerization, which is described in the appended examples.
It is also particularly within the scope of the present invention that the transformation of isoforms of A2E or derivatives from the non-apoptosis-inducing pro-form to the apoptosis-inducing compound may result in disruption of the bond resulting in a release of said compound from the above referred ligand which specifically binds to/interacts with a cell surface molecule, a molecule in the cytosol of a cell or a molecule specific for mitochondria.

As noted herein above the pharmaceutical composition of the invention may be administered systemically. Alternatively, the pharmaceutical composition of the invention may be administered locally.
Different routes of administration are described herein above.

A further embodiment of the invention relates to an antidote to a pharmaceutical composition of the invention which inactivates A2E, isoforms or derivatives thereof, comprising phospholipids and optionally further comprising suitable formulations of carrier, stabilizers and/or excipients and wherein said antidote is to be administered subsequently to said pharmaceutical composition.
It has been further surprisingly found in accordance with the invention, that the active compound of the pharmaceutical composition of the invention may be inactivated by an antidot. Said antidote allows the inactivation of the active compound after the completion of the treatment of an individual with the pharmaceutical compositions of the invention. Thus, said active compound does not remain active in the cells, tissues, organs or the complete body of an individual after the completion of a treatment until it is metabolically abolished by the individual.
The antidote of the invention may be administered in the same formulations and via the same routes as the pharmaceutical composition of the invention. The ratio in the concentration of the pharmaceutical composition and the antidote may be about 1:1 in case of the same formulation and the same route of administration of the antidote and the pharmaceutical composition.
The antidote of the invention may be formulated and administered as described for pharmaceutical compositions of the invention herein above.
Generally, the regimen as a regular administration of the antidote should be in the range of 0,1 µg to 10 mg units per day. If the regimen is a continuous infusion, it should also be in the range of 0,1 µg to 10 mg units per kilogram of body weight per minute, respectively. However, a more preferred dosage for continuous infusion might be in the range of 0.001 µg to 10 mg units per kilogram of body weight per hour. Progress can be monitored by periodic assessment.
Examples for phospholipids which may be comprised in the antidote of the invention comprise phosphatidylglycerin, 1,2-Dioleayl-sn-glycero-3-phosphatidylcoline (DOPC), 1,2-dilauroyl-sn-glycero-3-phosphatidylcoline (DLPC), 1-palmitoyl-2-oleoyl-sn-glycero-3-phosphatidylcoline (POPC), 1,2-dimyristoyl-sn-glycero-3-phosphocoline (DMPC), 1,2-dioleoyl-sn-glycero-3-phophoethanolamine (DOPE), 1,2- dipalmitoyl-sn-glycero-3-phosphocoline (DPPC), 1,2-distearoyl-sn-glycero-3-phophoethanolamine (DSPE), 1,2- dimyristoyl-sn-glycero-3-phosphoglycerol (DMPG) and 1,2-distearoyl-sn-glycero-3-phosphocoline (DSPC).
Preferably, said phospholipid is phosphatidylglycerin.

An alternative embodiment of the present invention is the use of N-retinyl-N-retinylidene ethanolamine (A2E), isoforms of A2E or derivatives thereof which induce apoptosis by detaching pro-apoptotic proteins from mitochondria for the preparation of a pharmaceutical composition for the treatment of proliferative diseases such as cancer diseases or psoriasis diseases.
The diseases to be treated by the recited pharmaceutical composition have been characterized and defined in more detail herein above.
In line with this embodiment of the invention, said pharmaceutical composition further comprises suitable formulations of carrier, stabilizers and/or excipients.

It is further envisaged by the use of the invention that said A2E, isoforms of A2E or derivatives thereof is/are linked to a ligand which specifically binds to/interacts with a cell surface molecule, a molecule in the cytosol of a cell or a molecule specific for mitochondria.
The terms "ligand", "specific binding to/interaction with", "cell surface molecule", "molecule in the cytosol of a cell" and "molecule specific for mitochondria" have been defined herein above.
Preferably, said cell surface molecule is a tumor marker and/or a molecule which is expressed by tumor and non-tumor cells but accessibly presented to the immune system only by tumor cells.
More preferably, said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

It is particularly envisaged by the use of the invention that said A2E, isoforms of A2E or derivatives thereof and said ligand are linked by a covalent bond
The use of the invention also comprises that said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.
It is also particularly within the scope of the use of the invention that the transformation of isoforms of A2E or derivatives from the non-apoptosis-inducing pro-form to the apoptosis-inducing compound may result in disruption of the bond resulting in a release of said compound from the above referred ligand which specifically binds to/interacts with a cell surface molecule, a molecule in the cytosol of a cell or a molecule specific for mitochondria.
The composition according to the use of the invention is to be administered systemically. Alternatively, said composition is to be administered locally.

One further embodiment of the invention relates to the use of one or more phospholipid/s for the preparation of an antidote to the pharmaceutical composition of the invention for the treatment of proliferative diseases inactivates A2E, isoforms or derivatives thereof and wherein said antidote is to be administered subsequently to said pharmaceutical composition.
Preferably, said antidote further comprises suitable formulations of carrier, stabilizers and/or excipients.
Examples for phospholipids which may be comprised in the antidote of the invention comprise phosphatidylglycerin, 1,2-Dioleayl-sn-glycero-3-phosphatidylcoline (DOPC), 1,2-dilauroyl-sn-glycero-3-phosphatidylcoline (DLPC), 1-palmitoyl-2-oleoyl-sn-glycero-3-phosphatidylcoline (POPC), 1,2-dimyristoyl-sn-glycero-3-phosphocoline (DMPC), 1,2-dioleoyl-sn-glycero-3-phophoethanolamine (DOPE), 1,2- dipalmitoyl-sn-glycero-3-phosphocoline (DPPC), 1,2-distearoyl-sn-glycero-3-phophoethanolamine (DSPE), 1,2- dimyristoyl-sn-glycero-3-phosphoglycerol (DMPG) and 1,2-distearoyl-sn-glycero-3-phosphocoline (DSPC).
It is envisaged that said phospholipid is phosphatidylglycerin.

A further embodiment of the invention relates to a method for the treatment of proliferative diseases such as cancer diseases or psoriasis diseases, the method comprising the step of administering N-retinyl-N-retinylidene ethanolamine (A2E), isoforms of A2E or derivatives thereof which induce apoptosis by detaching pro-apoptotic proteins from mitochondria.
The diseases to be treated by the recited pharmaceutical composition have been characterized and defined in more detail herein above.
Preferably, said A2E, isoforms of A2E or derivatives thereof is administered in combination with suitable formulations of carrier, stabilizers and/or excipients to a person in the need thereof.
It is envisaged by the method of the invention that said A2E, isoforms of A2E or derivatives thereof is/are linked to a ligand which specifically binds to/interacts with a cell surface molecule, a molecule in the cytosol of a cell or a molecule specific for mitochondria.
The terms "ligand", "specific binding to/interaction with", "cell surface molecule", "molecule in the cytosol of a cell" and "molecule specific for mitochondria" have been defined herein above.
Preferably, said cell surface molecule is a tumor marker and/or a molecule which is expressed by tumor and non-tumor cells but accessibly presented to the immune system only by tumor cells.
Further preferably, said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

According to the method of the invention said A2E, isoforms of A2E or derivatives thereof and said ligand may be linked by a covalent bond.
In one embodiment of the method of the invention said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.
It is also particularly within the scope of the method of the invention that the transformation of isoforms of A2E or derivatives from the non-apoptosis-inducing pro-form to the apoptosis-inducing compound may result in disruption of the bond resulting in a release of said compound from the above referred ligand which specifically binds to/interacts with a cell surface molecule, a molecule in the cytosol of a cell or a molecule specific for mitochondria.
The method of the invention may comprise a systemic administration of the composition.
Alternatively said composition is administered locally.

It is particularly envisaged by the method of the invention that said method is part of a combination of different methods for the treatment of proliferative diseases.

In one embodiment the method of the invention further comprises a step administering an antidote of the composition subsequent of the step of administration of said composition to inactivate A2E, isoforms or derivatives thereof.
Said antidote may in line with the invention further comprises suitable formulations of carrier, stabilizers and/or excipients.

Preferably, the active compound of the antidote which inactivates A2E, isoforms or derivatives thereof is a phospholipid.
Examples for phospholipids which may be comprised in the antidote of the invention comprise phosphatidylglycerin, 1,2-Dioleayl-sn-glycero-3-phosphatidylcoline (DOPC), 1,2-dilauroyl-sn-glycero-3-phosphatidylcoline (DLPC), 1-palmitoyl-2-oleoyl-sn-glycero-3-phosphatidylcoline (POPC), 1,2-dimyristoyl-sn-glycero-3-phosphocoline (DMPC), 1,2-dioleoyl-sn-glycero-3-phophoethanolamine (DOPE), 1,2- dipalmitoyl-sn-glycero-3-phosphocoline (DPPC), 1,2-distearoyl-sn-glycero-3-phophoethanolamine (DSPE), 1,2- dimyristoyl-sn-glycero-3-phosphoglycerol (DMPG) and 1,2-distearoyl-sn-glycero-3-phosphocoline (DSPC).
More preferably, said phospholipid is phosphatidylglycerin.

A further alternative embodiment of the invention relates to a screening method for the identification of new isoforms of A2E or derivatives thereof which induce apoptosis by detaching pro-apoptotic proteins from mitochondria, said method comprising the steps of:
(a) contacting a cell or tissue with an isoform of A2E or a derivative thereof; and
(b) detecting whether said isoform of A2E or said derivative thereof induces apoptosis in the cell or tissue.
Methods for the detection of the induction of apoptosis in a cell are, as noted herein above, known in the art. Possible approaches for screening methods according to the present invention are described in the appended examples which are not to be construed to be limiting in this regard.
The recited cells may be porcine primary retinal pigment epithelial cells (RPE) or cell lines e.g. selected from ARPE-19 and MDCK-F1 cells Preferably, said cell or tissue is a tumor cell or tissue.
More preferably, said cell is a ARPE-19 cell, a MDCK-F1 cell or a HeLa cell.
The method of the invention may further comprise the step of transforming the isoform of A2E or a derivative from a pro-form to apoptosis-inducing compound by electromagnetic irradiation, preferably by light, more preferably by blue light.

In one embodiment of the screening method of the invention said method comprises the additional step of detecting the efficiency of antidots to inhibit the induction of apoptosis of the isoform of A2E or a derivative, wherein said antidots comprise phospholipids.
The decrease or absence of apoptosis in the cells or tissue which are contacted in step (a) of the screening method of the invention with an isoform of A2E or a derivative thereof which has been identified to induce apoptosis by detaching pro-apoptotic proteins from mitochondria is characteristic for the efficiency of the antidot.
Preferably, said phospholipid is phosphatidylglycerin.

The present invention also relates to a pharmaceutical composition comprising an isoform of A2E or a derivative identified by the screening method of the invention, and optionally further comprising suitable formulations of carrier, stabilizers and/or excipients.
Furthermore, the invention relates to the use of an isoform of A2E or a derivative identified by the screening method of the invention, for the preparation of a pharmaceutical composition for the treatment of proliferative diseases, cancer diseases or psoriasis diseases, wherein said pharmaceutical composition optionally further comprising suitable formulations of carrier, stabilizers and/or excipients.
The present invention also envisages a method for the treatment of proliferative diseases, cancer diseases or psoriasis diseases, the method comprising the step of administering isoforms of A2E or derivatives thereof which have been identified by the screening method of the invention, wherein said pharmaceutical composition optionally further comprising suitable formulations of carrier, stabilizers and/or excipients.

Said pharmaceutical composition, use or method particularly comprise embodiment, wherein said isoform of A2E or derivative thereof is linked to a ligand which specifically binds to/interacts with a cell surface molecule, a molecule in the cytosol of a cell or a molecule specific for mitochondria.
The terms "ligand", "specific binding to/interaction with", "cell surface molecule", "molecule in the cytosol of a cell" and "molecule specific for mitochondria" have been defined herein above.

Preferably, said cell surface molecule is a tumor marker, tumor associated antigen and/or a molecule which is expressed by tumor and non-tumor cells but accessibly presented to the immune system only by tumor cells.
Further preferably, said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

Also comprised by the invention are pharmaceutical compositions, the uses or the methods, wherein said isoform of A2E or derivative thereof and said ligand are linked by a covalent bond.

Furthermore, the invention particularly comprises above described pharmaceutical compositions, the uses or the methods, wherein said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.
It is also particularly within the scope of the pharmaceutical compositions, the uses or the methods, of the invention that the transformation of isoforms of A2E or derivatives from the non-apoptosis-inducing pro-form to the apoptosis-inducing compound may result in disruption of the bond resulting in a release of said compound from the above referred ligand which specifically binds to/interacts with a cell surface molecule, a molecule in the cytosol of a cell or a molecule specific for mitochondria.

It is preferred for said pharmaceutical compositions, the uses or the methods, that said composition is to be administered systemically.
Alternatively, said composition is to be administered locally.

It is also envisaged, that the above described method may be part of a combination of different methods for the treatment of proliferative diseases.

An alternative embodiment according to the present invention relates to a use of phospholipids for the preparation of an antidote to a pharmaceutical composition for the treatment of proliferative diseases as defined herein above, which inactivates A2E, isoforms or derivatives thereof and wherein said antidote is to be administered subsequently to said pharmaceutical composition.
It is preferred, that said antidote further comprises suitable formulations of carrier, stabilizers and/or excipients.

Further envisaged by the present invention is an above described method, wherein subsequent of administration of said composition an antidote is administered to inactivate said isoforms of A2E or derivatives thereof.
Said antidote may further comprises suitable formulations of carrier, stabilizers and/or excipients.
Preferably, the active compound of the antidote which inactivates A2E, isoforms or derivatives thereof is a phospholipid.

More preferably, said phospholipid recited in the context of the above described use or method is phosphatidylglycerin.

In a further alternative embodiment the invention provides a screening method for the identification of new compounds for antidote for pharmaceutical compositions of the invention, said method comprising the steps of:
(a) contacting in a first experimental setting a cell or tissue with A2E, an isoform of A2E or a derivative thereof which induces apoptosis by detaching pro-apoptotic proteins from mitochondria;
(b) contacting in a second experimental setting a cell or tissue with A2E, an isoform of A2E or a derivative thereof which induces apoptosis by detaching pro-apoptotic proteins from mitochondria and a candidate compound;
(c) detecting whether the presence of said compound in setting (b) decreases the apoptosis in the cell or tissue compared to setting (a), wherein a decrease or an absence of apoptosis in setting (b) is indicative for efficiency of the antidote.

Preferably, said cell or tissue is a tumor cell or tissue.
It is particularly envisaged that said cell is a ARPE-19 cell, a MDCK-F1 cell or a HeLa cell.

It is also envisaged by the invention to use of a compound identified by the screening method for the identification of new compounds for antidote for pharmaceutical compositions of the invention for the preparation of an antidote to a pharmaceutical composition for the treatment of proliferative diseases described herein above. Said antidote is to be administered subsequently to said pharmaceutical composition.
Furthermore, the method of treatment of the invention may comprise the subsequent of administration of a compound identified by the screening method for the identification of new compounds for antidotes, which is administered to inactivate said A2E, isoforms of A2E or derivatives thereof.
In a preferred embodiment of said use and said method of treatment the antidote may further comprises suitable formulations of carrier, stabilizers and/or excipients

The Figures show:
**Figure 1:**
   Figure 1 depicts structures of A2E and iso-A2E.
**Figure 2:**
   Figure 2 depicts a schema of the visual cycle, highlighting the metabolism of retinal in rod outer segments (ROS) and RPE cells.
**Figure 3:**

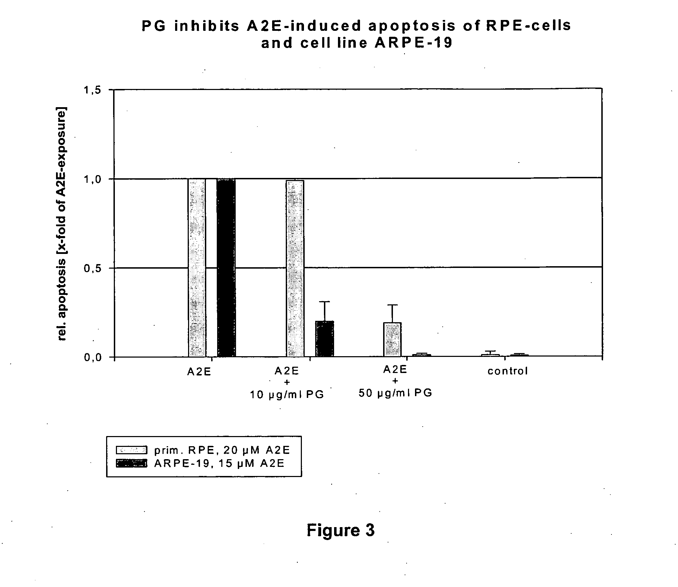

   Figure 3 depicts the result of an experiment demonstrating the protective effect of a phospholipid (PG) regarding the apoptotic effect of A2E on different cell types. RPE, ARPE-19, and MDCK-F1 cells are exposed to both PG (0, 10, 50 µg/ml) and A2E (0, 15 or 20 µM) for 4 hrs. Then they are washed 3 times with PBS and the apoptosis is determined by Cell Death ELISA Plus Kit (Roche, Mannheim, Germany).
**Figure 4:**

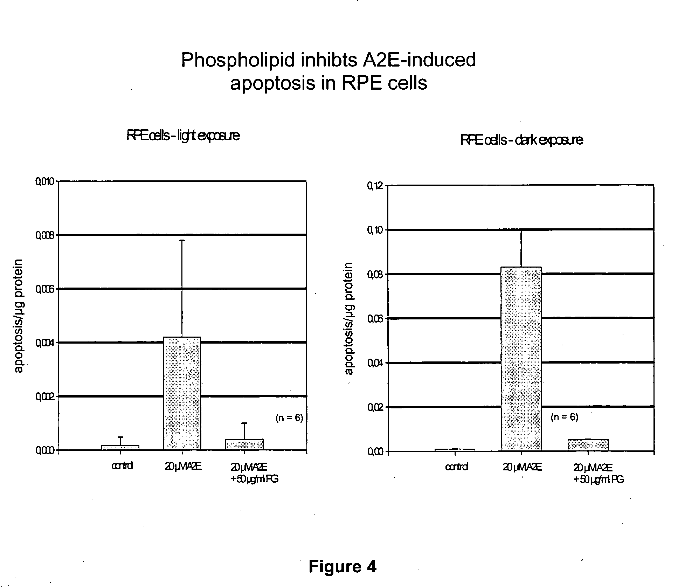

   Figure 4 depicts the result of an experiment demonstrating the protective effect of a phospholipid (PG) regarding the apoptotic effect of photo-isomerization of iso-A2E on different cell types. RPE, ARPE-19, and MDCK-F1 cells are exposed to both PG (0, 10, 50 µg/ml) and iso-A2E (0, 15 or 20 µM) under the influence of light for 4 hrs. Then they are washed 3 times with PBS and the apoptosis is determined by Cell Death ELISA Plus Kit (Roche, Mannheim, Germany)

The invention will now be described by reference to the following biological examples which are merely illustrative and are not to be construed as a limitation of scope of the present invention.

### Examples

As an example, the apoptotic effect of A2E on porcine primary retinal pigment epithelial cells (RPE), and the cell lines ARPE-19 and MDCK-F1 cells is analyzed.
Example 1 describes the apoptotic effect of A2E on RPE, ARPE-19, and MDCK-F1 cells. The cells are exposed to 0, 15 and 30 µM of A2E for 2, 4, 6 and 22 hrs. Then they are washed 3 times with PBS and the apoptosis is determined by Cell Death ELISA Plus Kit (Roche, Mannheim, Germany).
Example 2a describes the protective effect of a phospholipid (PG) regarding the apoptotic effect of A2E on different cell types. RPE, ARPE-19, and MDCK-F1 cells are exposed to both PG (0, 10, 50 µg/ml) and A2E (0, 15 or 20 µM) for 4 hrs. Then they are washed 3 times with PBS and the apoptosis is determined by Cell Death ELISA Plus Kit (Roche, Mannheim, Germany) (see figure 3).
Example 2b describes the protective effect of a phospholipid (PG) regarding the apoptotic effect of A2E on different cell types. RPE, ARPE-19, and MDCK-F1 cells are exposed to A2E (0, 15 or 20 µM) for 17 hrs and finally they are exposed to both PG (0, 10, 50 µg/ml) and A2E (0, 15 or 20 µM) for additional 4 hrs. Then they are washed 3 times with PBS and the apoptosis is determined by Cell Death ELISA Plus Kit (Roche, Mannheim, Germany).
Example 2c describes the protective effect of a phospholipid (PG) regarding the apoptotic effect of A2E on different cell types. RPE, ARPE-19, and MDCK-F1 cells are exposed to PG (0, 10, 50 µg/ml) for 17 hrs and finally they are exposed to both, PG (0, 10, 50 µg/ml) and A2E (0, 15 or 20 µM) for additional 4 hrs. Then they are washed 3 times with PBS and the apoptosis is determined by Cell Death ELISA Plus Kit (Roche, Mannheim, Germany).
Example 3 describes the apoptotic effect of A2E on RPE, ARPE-19, and MDCK-F1 cells after application of iso-A2E and exposure in light (photo-isomerization). The cells are exposed to 0, 20 and 40 µM of iso-A2E under the influence of light for 2, 4, 6 and 22 hrs. Then they are washed 3 times with PBS and the apoptosis is determined by Cell Death ELISA Plus Kit (Roche, Mannheim, Germany).
Example 4 describes the protective effect of a phospholipid (PG) regarding the apoptotic effect of photo-isomerization of iso-A2E on different cell types. RPE, ARPE-19, and MDCK-F1 cells are exposed to both PG (0, 10, 50 µg/ml) and iso-A2E (0, 15 or 20 µM) under the influence of light for 4 hrs. Then they are washed 3 times with PBS and the apoptosis is determined by Cell Death ELISA Plus Kit (Roche, Mannheim, Germany) (see figure 4).

### Material:

*A2E- and iso-A2E-synthesis*: A2E was synthesized from all-trans-retinal and ethanolamine and purified by silica gel chromatography plates according to Parish *et al*. and Suter *et al*. [1; 2]. A2E and iso-A2e were separated by HPLC as described by Parish et al. [1].

*Phospholipid:* the PG (L-A-Phosphatidyl-DL-Glycerol sodium # P8318) was purchased from Sigma-Aldrich, Schnelldorf, Germany.
Cell culture media and FCS were purchased from Cambrex Bio Science, Verviers, Belgium.

### Methods:

### Cell culture:

RPE and ARPE-19 cells: Primary porcine retinal pigment epithelial cells were isolated as previously described [3]. RPE and ARPE-19 cells [4] were grown to confluence in DMEM-F12 medium supplemented with 10 % FCS, 124 mmol/l HCO₃⁻, 5,37 g/l HEPES in a humidified 5 % CO₂ atmosphere for 1 (ARPE 19) or 2 (RPE) weeks. Confluent monolayers were split by trypsinization (0.25 g/l) in Ca²⁺- and Mg²⁺- free, EDTA-containing Ringer's solution. The experiments were done after culturing the cells in 96-well tissue culture plates using DMEM-F12 medium supplemented with 2 % FCS for one week.
MDCK-F1 cells were grown to confluence in MEM medium supplemented with 10 % FCS, 124 mmol/l HCO₃⁻, 5,37 g/l HEPES in a humidified 5 % CO₂ atmosphere for one week [5]. Confluent monolayers were split by trypsinization (0.25 g/l) in Ca²⁺- and Mg²⁺-free, EDTA-containing Ringer's solution. The experiments were done after culturing the cells in 96-well tissue culture plates using MEM medium supplemented with 10 % FCS for one week.
*Detection of apoptosis*: Apoptosis was detected by Cell Death ELISA Plus Kit (Roche, Mannheim, Germany). The cell lysis was done with an alternative buffer (PBS + 0,1 % v/v Triton-X100). For normalization of the apoptotic fluorescence signal, the content of total protein was determined by BCA-Assay (Micro BCA Kit, Perbio Science, Bonn, Germany) and the apoptosis was expressed as apoptotic fluorescence signal/µg total protein.

### Literature:

1. Parish CA, Hashimoto M, Nakanishi K, Dillon J, Sparrow J.lsolation and one-step preparation of A2E and iso-A2E, fluorophores from human retinal pigment epithelium. Proc Natl Acad Sci U S A. 1998 Dec 8;95(25):14609-13.
2. Suter M, Reme C, Grimm C, Wenzel A, Jaattela M, Esser P, Kociok N, Leist M, Richter C.Age-related macular degeneration. The lipofusion component N-retinyl-N-retinylidene ethanolamine detaches proapoptotic proteins from mitochondria and induces apoptosis in mammalian retinal pigment epithelial cells. J Biol Chem. 2000 Dec 15;275(50):39625-30.
3. Mannerstrom M, Maenpaa H, Toimela T, Salminen L, Tahti H.The phagocytosis of rod outer segments is inhibited by selected drugs in retinal pigment epithelial cell cultures. Pharmacol Toxicol. 2001 Jan;88(1):27-33.
4. Dunn KC, Aotaki-Keen AE, Putkey FR, Hjelmeland LM. ARPE-19, a human retinal pigment epithelial cell line with differentiated properties. Exp Eye Res. 1996 Feb;62(2):155-69.
5. Schwab A, Geibel J, Wang W, Oberleithner H, Giebisch G. Mechanism of activation of K+ channels by minoxidil-sulfate in Madin-Darby canine kidney cells. J Membr Biol. 1993 Mar; 132(2): 125-36.

Eldred, G.E., and Lasky M.R. (1993) Retinal age pigments generated by self-assembling lysosomotropic detergents. Nature 361, 724-726.
Hengartner, M.O. (2000) The biochemistry of apoptosis. Nature 407, 770-776.
Mata, N.L., Weng, J., and Travis, G.H. (2000) Biosynthesis of a major lipofuscin fluorophore in mice and humans with ABCR-mediated retinal and macular degeneration. Proc. Natl. Acad. Sci. USA. 97, 7154-7159.
Parish, C.A., Hashimoto, M., Nakanishi, K., Dillon, J., and Sparrow, J.R. (1998) Isolation and one-step preparation of A2E and iso-A2E, fluorophores from human retinal pigment epithelium. Proc. Natl. Acad. Sci. USA. 95, 14609-14613.
Richter, C. (2001) Insight into age-related macular degeneration: New vision in sight. Cell Death Differ. 8, 207-209.
Richter, C. (1993) Pro-oxidants and mitochondrial Ca²⁺: their relationship to apoptosis and oncogenesis. FEBS Lett. 325, 104-107.
Sparrow, J.R., and Cai, B. (2001) Blue light-induced apoptosis of A2E-containing RPE: involvement of caspase-3 and protection by Bcl-2. Invest. Ophthalmol. Vis. Sci. 42, 1356-1362.
Sparrow, J.R., Nakanishi, K., and Parish, C.A. (2000) The lipofuscin fluorophore A2E mediates blue light-induced damage to retinal pigmented epithelial cells. Invest. Ophthalmol. Vis. Sci. 41, 1981-1989.
Shaban, H., and Richter, C. (2002) A2E and blue light in the retina: The paradigm of age-related macular degeneration. Biol. Chem. 383, 537-545.
Shaban, H., Borras, C., Vina, J., and Richter, C. (2002) Phosphatidylglycerol potently protects human retinal pigment epithelial cells against apoptosis induced by A2E, a compound suspected to cause age-related macula degeneration. Exp. Eye Res. 75, 99-108.
Shaban, H., Gazzotti, P., and Richter, C. (2001) Cytochrome c oxidase inhibition by N-retinyl-N-retinylidene ethanolamine, a compound suspected to cause age-related macula degeneration. Arch. Biochem. Biophys. 394, 111-116.
Suter, M., Reme, C., Grimm, C., Wenzel, A., Jaattela, M., Esser, P., Kociok, N., Leist, M., and Richter, C. (2000) Age-related macular degeneration. The lipofuscin component N-retinyl-N-retinylidene ethanolamine detaches proapoptotic proteins from mitochondria and induces apoptosis in mammalian retinal pigment epithelial cells. J. Biol. Chem. 275, 39625-39630.
Wallace, D.C. (1999) Mitochondrial diseases in man and mouse. Science 283, 1482-1488.

## Claims

**1.** A pharmaceutical composition comprising N-retinyl-N-retinylidene ethanolamine (A2E), isoforms of A2E or derivatives thereof which induce apoptosis by detaching pro-apoptotic proteins from mitochondria and optionally further comprising suitable formulations of carrier, stabilizers and/or excipients.

**2.** The pharmaceutical composition according to claim 1, wherein said A2E, isoforms of A2E or derivatives thereof is/are linked to a ligand which specifically binds to/interacts with a cell surface molecule, a molecule in the cytosol of a cell or a molecule specific for mitochondria.

**3.** The pharmaceutical composition according to claim 2, wherein said cell surface molecule is a tumor marker, tumor associated antigen and/or a molecule which is expressed by tumor and non-tumor cells but accessibly presented to the immune system only by tumor cells.

**4.** The pharmaceutical composition according to claim 3, wherein said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

**5.** The pharmaceutical composition according to any of claims 2 to 4, wherein said A2E, isoforms of A2E or derivatives thereof and said ligand are linked by a covalent bond.

**6.** The pharmaceutical composition according to any of claims 1 to 5, wherein said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.

**7.** The pharmaceutical composition according to any of claims 1 to 6, wherein said composition is administered systemically.

**8.** The pharmaceutical composition according to any of claims 1 to 6, wherein said composition is administered locally.

**9.** An antidote to a pharmaceutical composition according to any of claims 1 to 8 which inactivates A2E, isoforms or derivatives thereof, comprising phospholipids and optionally further comprising suitable formulations of carrier, stabilizers and/or excipients and wherein said antidote is to be administered subsequently to said pharmaceutical composition.

**10.** The antidote according to claim 9, wherein said phospholipid is phosphatidylglycerin.

**11.** Use of N-retinyl-N-retinylidene ethanolamine (A2E), isoforms of A2E or derivatives thereof which induce apoptosis by detaching pro-apoptotic proteins from mitochondria for the preparation of a pharmaceutical composition for the treatment of proliferative diseases, cancer diseases or psoriasis diseases.

**12.** The use according to claim 11, wherein said pharmaceutical composition further comprises suitable formulations of carrier, stabilizers and/or excipients

**13.** The use according to claim 11 or 12, wherein said A2E, isoforms of A2E or derivatives thereof is/are linked to a ligand which specifically binds to/interacts with a cell surface molecule, a molecule in the cytosol of a cell or a molecule specific for mitochondria.

**14.** The use according to claim 13, wherein said cell surface molecule is a tumor marker and/or a molecule which is expressed by tumor and non-tumor cells but accessibly presented to the immune system only by tumor cells.

**15.** The use according to claim 14, wherein said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

**16.** The use according to any of claims 13 to 15, wherein said A2E, isoforms of A2E or derivatives thereof and said ligand are linked by a covalent bond.

**17.** The use according to any of claims 13 to 16, wherein said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.

**18.** The use according to any of claims 11 to 17, wherein said composition is administered systemically.

**19.** The use according to any of claims 11 to 17, wherein said composition is administered locally.

**20.** Use of phospholipids for the preparation of an antidote to a pharmaceutical composition for the treatment of proliferative diseases as defined in any of claims 13 to 22 which inactivates A2E, isoforms or derivatives thereof and wherein said antidote is to be administered subsequently to said pharmaceutical composition.

**21.** The use according to claim 20, wherein said antidote further comprises suitable formulations of carrier, stabilizers and/or excipients.

**22.** The use according to claim 20 or 21, wherein said phospholipid is phosphatidylglycerin.

**23.** A method for the treatment of proliferative diseases, cancer diseases or psoriasis diseases, the method comprising the step of administering N-retinyl-N-retinylidene ethanolamine (A2E), isoforms of A2E or derivatives thereof which induce apoptosis by detaching pro-apoptotic proteins from mitochondria.

**24.** The method according to claim 23, wherein said A2E, isoforms of A2E or derivatives thereof is administered in combination with suitable formulations of carrier, stabilizers and/or excipients to a person in the need thereof.

**25.** The method according to claim 23 or 24, wherein said A2E, isoforms of A2E or derivatives thereof is/are linked to a ligand which specifically binds to/interacts with a cell surface molecule, a molecule in the cytosol of a cell or a molecule specific for mitochondria.

**26.** The method according to claim 25, wherein said cell surface molecule is a tumor marker and/or a molecule which is expressed by tumor and non-tumor cells but accessibly presented to the immune system only by tumor cells.

**27.** The method according to claim 26, wherein said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

**28.** The method according to any of claims 25 to 27, wherein said A2E, isoforms of A2E or derivatives thereof and said ligand are linked by a covalent bond.

**29.** The method according to any of claims 23 to 28, wherein said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.

**30.** The method according to any of claims 23 to 29, wherein said composition is administered systemically.

**31.** The method according to any of claims 23 to 29, wherein said composition is administered locally.

**32.** The method according to any of claims 23 to 30, which is part of a combination of different methods for the treatment of proliferative diseases.

**33.** The method according to any of claims 23 to 32, wherein subsequent of administration of said composition an antidote is administered to inactivate A2E, isoforms or derivatives thereof.

**34.** The method according to claim 33, wherein said antidote further comprises suitable formulations of carrier, stabilizers and/or excipients.

**35.** The method according to claim 33 or 34, wherein the active compound of the antidote which inactivates A2E, isoforms or derivatives thereof is a phospholipid.

**36.** The method according to claim 36, wherein said phospholipid is phosphatidylglycerin.

**37.** A screening method for the identification of new isoforms of A2E or derivatives thereof which induce apoptosis by detaching pro-apoptotic proteins from mitochondria, said method comprising the steps of:
(a) contacting a cell or tissue with an isoform of A2E or a derivative thereof; and
(b) detecting whether said isoform of A2E or said derivative thereof induces apoptosis in the cell or tissue.

**38.** The method of claim 37, wherein said cell or tissue is a tumor cell or tissue.

**39.** The method of claim 38, wherein said cell is a ARPE-19 cell, a MDCK-F1 cell or a HeLa cell.

**40.** The method of any of claims 37 to 39, further comprising the step of transforming the isoform of A2E or a derivative from a pro-form to apoptosis-inducing compound by electromagnetic irradiation, preferably by light, more preferably by blue light.

**41.** The method of any of claims 37 to 40, further comprising the step detecting the efficiency of an antidots for the induction of apoptosis, wherein said antidots comprise phospholipids.

**42.** The method of claim 41, wherein said phospholipid is phosphatidylglycerin.

**43.** A pharmaceutical composition comprising an isoform of A2E or a derivative identified by a method according to any of claims 37 to 41, and optionally further comprising suitable formulations of carrier, stabilizers and/or excipients.

**44.** Use of an isoform of A2E or a derivative identified by a method according to any of claims 37 to 41, for the preparation of a pharmaceutical composition for the treatment of proliferative diseases, cancer diseases or psoriasis diseases, wherein said pharmaceutical composition optionally further comprising suitable formulations of carrier, stabilizers and/or excipients.

**45.** A method for the treatment of proliferative diseases, cancer diseases or psoriasis diseases, the method comprising the step of administering isoforms of A2E or derivatives thereof which have been identified by a method according to any of claims 37 to 41, wherein said pharmaceutical composition optionally further comprising suitable formulations of carrier, stabilizers and/or excipients.

**46.** The pharmaceutical composition according to claim 43, the use according to claim 44 or the method according to claim 45 wherein said isoform of A2E or derivative thereof is linked to a ligand which specifically binds to/interacts with a cell surface molecule, a molecule in the cytosol of a cell or a molecule specific for mitochondria.

**47.** The pharmaceutical composition, the use or the method according to claim 46, wherein said cell surface molecule is a tumor marker, tumor associated antigen and/or a molecule which is expressed by tumor and non-tumor cells but accessibly presented to the immune system only by tumor cells.

**48.** The pharmaceutical composition, the use or the method according to claim 47, wherein said tumor marker, tumor associated antigen and/or molecule which is accessibly presented only by tumor cells is selected from the group consisting of CD5, CD19, CD20, CD25, CD30, CD33, CD44v6, CD63, CCR5, CCR8, βhCG, β-catenin, A33 Antigen, CA19-9 marker, CA-125 marker, Carboanhydrase IX (MN/CA IX), CEA, Cyclin-dependent kinase 4, desmoglein 4, E-cadherin neoepitope, endosialin, EGFRvIII, EGFR, ErbB-2, EphA2, EpCAM, FAP (fibroblast activation antigen), Fetal Acetylcholine Receptor, ganglioside GD3, 9-O-Acetyl-GD3, GM2, Globo H, fucosyl GM1, GD2, HER-2 neu, HER-3, HER-4, HPV E6, HPV E7, Lewis-Y, LG, Ly-6; MUC-1 (mucin), MUC2, MUC3, MUC4, MUC5AC, MUC5B, MUC7, MAGE-1, MAGE-3, Muellerian Inhibitory Substance (MIS) Receptor type II, Melanoma Chondroitin Sulfate Proteoglycan (MCSP), mesothelin, (membrane-bound) IgE, Poly SA, PSMA, Prostate Stem Cell Antigen (PSCA), p53 (Tumor suppressor p53), Plasma Cell Antigen, Sonic Hedgehog (Shh), STEAP, SAS, sTn (sialylated Tn antigen; TAG-72), TNF-alpha precursor, Tyrosinase, and Wue-1.

**49.** The pharmaceutical composition, the use or the method according to any of claims 44 to 48, wherein said isoform of A2E or derivative thereof and said ligand are linked by a covalent bond.

**50.** The pharmaceutical composition pharmaceutical composition, the use or the method according to any of claims 43 to 49, wherein said pharmaceutical composition comprises isoforms of A2E or derivatives thereof which are non-apoptosis-inducing pro-forms and wherein said pharmaceutical composition is to be administered to a person in the need thereof and the pro-forms of said isoforms of A2E or derivatives thereof are transformed to apoptosis-inducing compound by electromagnetic irradiation, preferably by light, more preferably by blue light.

**51.** The pharmaceutical composition pharmaceutical composition, the use or the method according to any of claims 43 to 50, wherein said composition is administered systemically.

**52.** The pharmaceutical composition pharmaceutical composition, the use or the method according to any of claims 43 to 51, wherein said composition is administered locally.

**53.** The method according to any of claims 45 to 52, which is part of a combination of different methods for the treatment of proliferative diseases.

**54.** Use of phospholipids for the preparation of an antidote to a pharmaceutical composition for the treatment of proliferative diseases as defined in any of claims 43 to 52 which inactivates A2E, isoforms or derivatives thereof and wherein said antidote is to be administered subsequently to said pharmaceutical composition.

**55.** The use according to claim 54 wherein said antidote further comprises suitable formulations of carrier, stabilizers and/or excipients.

**56.** The method according to any of claims 45 to 53, wherein subsequent of administration of said composition an antidote is administered to inactivate said isoforms of A2E or derivatives thereof.

**57.** The method according to claim 56, wherein said antidote further comprises suitable formulations of carrier, stabilizers and/or excipients.

**58.** The method according to claim 56 or 57, wherein the active compound of the antidote which inactivates A2E, isoforms or derivatives thereof is a phospholipid.

**59.** The use according to claim 54 to 55 or the method according to claim 58, wherein said phospholipid is phosphatidylglycerin.

**60.** A screening method for the identification of new compounds for antidote for pharmaceutical compositions as defined in any of claims 1 to 8 or 43, said method comprising the steps of:
(a) contacting in a first experimental setting a cell or tissue with A2E, an isoform of A2E or a derivative thereof which induces apoptosis by detaching pro-apoptotic proteins from mitochondria;
(b) contacting in a second experimental setting a cell or tissue with A2E, an isoform of A2E or a derivative thereof which induces apoptosis by detaching pro-apoptotic proteins from mitochondria and a candidate compound;
(c) detecting whether the presence of said compound in setting (b) decreases the apoptosis in the cell or tissue compared to setting (a), wherein a decrease or an absence of apoptosis in setting (b) is indicative for efficiency of the antidote.

**61.** The method of claim 60, wherein said cell or tissue is a tumor cell or tissue.

**62.** The method of claim 61, wherein said cell is a ARPE-19 cell, a MDCK-F1 cell or a HeLa cell.

**63.** Use of a compound identified by a method according to any of claims 60 to 62 for the preparation of an antidote to a pharmaceutical composition for the treatment of proliferative diseases as defined in any of claims 1 to 8 or 43 to 52 which inactivates A2E, isoforms or derivatives thereof and wherein said antidote is to be administered subsequently to said pharmaceutical composition.

**64.** The method according to any of claims 23 to 32 and 45 to 53, wherein subsequent of administration of said composition an antidote is administered to inactivate said A2E, isoforms of A2E or derivatives thereof.

**66.** The use according to claim 63 and the method according to claim 64, wherein said antidote further comprises suitable formulations of carrier, stabilizers and/or excipients.
